# EUROPEAN PATENT APPLICATION

(11) **EP 2 599 544 A1**
(43) Date of publication of application: **05.06.2013**
(21) Application number: 11191546.8
(22) Date of filing: 01.12.2011
(51) Int. Cl.: B01J 31/20, B01J 31/18, C07C 67/40

(54) **A process for producing alkyl esters by dehydrogenation of a primary alcohol using a homogenous catalyst system**

(71) Applicant: Leibniz-Institut für Katalyse e.V. an der Universität Rostock, 18059 Rostock (DE)
(72) Inventor: Nielsen, Martin, 4623 Lille Skensved (DK); Kammer, Anja, 18311 Ribnitz-Damgarten (DE); Junge, Henrik, 18147 Rostock (DE); Beller, Matthias, 18211 Neinhagen (DE)
(74) Representative: Gulde Hengelhaupt Ziebig & Schneider

(57) **Abstract**

The invention refers to a process for producing alkyl ester, especially for producing ethyl acetate, which comprises reacting a primary alcohol in the presence of a ruthenium-based homogeneous catalyst system containing a tridentate pincer ligand. A first homogenously catalyzed acceptorless dehydrogenation of alcohols to the corresponding alkyl esters was developed preferably the dehydrogenation of ethanol to ethyl acetate using a PNP pincer complex of ruthenium as the catalyst.

## Description

The invention refers to a process for producing alkyl esters, especially for producing ethyl acetate, which comprises reacting a primary alcohol in the presence of a ruthenium-based homogeneous catalyst system comprising a tridentate pincer ligand.

In 2008 the world market for ethyl acetate has been estimated to be 2.5M tons/a. This industrially highly important intermediate is one of the major derivatives of acetic acid. Apart from being used as solvent it is an important intermediate in the food industry and for various customer applications such as glues, inks, and perfumes. Today, its manufacture mainly relies on three main procedures: (1) the Fischer esterification reaction of ethanol and acetic acid, (2) the Tishchenko reaction by combination of two acetaldehyde molecules, and (3) the addition of acetic acid to ethylene. All these processes use petrochemical feedstock derived from primary fossil fuels, a limited and intrinsically polluting supply. With the growing awareness of more sustainable production and green chemistry alternative procedures employing renewable resources are of significant interest.

At present the synthesis of ethyl acetate from ethanol has been mainly studied applying heterogeneous catalysts with special focus on Cu-based catalysts. In general, yields up to 56% have been achieved. Obviously, using known hydrogen acceptors inherently result in low atom economy and high waste formation. Hence, regarding cost, such a procedure is not applicable.

Therefore, it is an object of the invention to find a catalyst system capable of transforming an alcohol to the corresponding alkyl ester under acceptorless and neat conditions. Especially it was an object of the present invention to find a catalyst system capable of transforming ethanol to ethyl acetate.

The present invention provides a production method for alkyl esters. A primary alcohol is used as a starting material to produce the corresponding alkyl ester with high yields by a simple process using a ruthenium-based homogenous catalyst system comprising a tridentate ligand. In an embodiment of the present invention ethanol is used to produce ethyl acetate. Notably, no solvent and no hydrogen acceptor are employed. Reports on acceptorless dehydrogenation of ethanol to form ethyl acetate are unknown.

According to the present invention, the alkyl ester can be obtained highly selectively by a simple process neither requiring a special mixing operation nor necessitating the operation of separating the catalyst.

The starting material alcohol used in the process for producing alkyl esters according to the present invention includes especially linear and branched saturated C2 to C18 primary alcohols and aralkyl alcohols, preferably linear alcohols. Specific examples include ethanol, 1-propanol, 1-butanol, 1-pentanol, 1-hexanol, 1-heptanol, 1-octanol, 1-nonanol, 1-decanol, 1-undecanol, 1-dodecanol, 1-tridecanol, 1-tetradecanol, 1-pentadecanol, 1-hexadecanol, 1-heptadecanol, 1-octadecanol.

Ethanol, 1-propanol, 1-butanol, 1-octanol and 1-octadecanol are especially preferred used alcohols. More preferred are ethanol and 1-octanol.

In a preferred embodiment of the invention the ruthenium-based catalyst system having a tridentate pincer ligand has the general formula **I**

RuXY (CO) L **I**,

wherein
X is hydride (H⁻) or halogen
Y is hydride (H⁻) or halogen
L is a tridentate pincer ligand selected from a PNP pincer type complex, a PNN pincer type complex, a PCP pincer type complex, a NCN pincer type complex, a PCN pincer type complex or NNN pincer type complex.

Halogen means F, Cl, Br, I, preferably Cl.

Tridentate pincer ligands are well known for a person skilled in the art and available commercially or may be synthesised according to methods well known. They are described for example in the following reviews: a) Johnson, T. C.; Morris, D. J.; Wills, R. Chem. Soc. Rev. 2010, 39, 81. b) Dobereiner, G. E.; Crabtree, R. H. Chem. Rev. 2010, 110, 681. c) Milstein, D. Top. Catal. 2010, 53, 915.

In an embodiment of the present invention the used catalyst of the present invention is a PNP pincer complex of ruthenium of the above formula I, wherein X and Y have the above meaning and

L has the following formula II 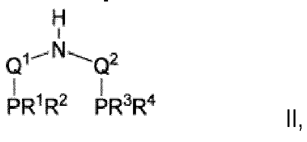 wherein
R¹, R², R³, R⁴ may be identical or different and represent hydrogen, alkyl, cycloalkyl, aryl, aralkyl, an alkyloxy group, a cycloalkyloxy group, an aryloxy group, aralkyloxy group, a heterocyclic group, amino group or substituted. R⁴ or R³ and R¹ and R², these are not to form a ring with the phosphorus atom adjacent to bond together may be.
Q¹ and Q² may be identical or different divalent alkylene (-CₙH₂ₙ-radicals) or alkenylene (-CₙHₙ-), which may have substituents, cycloalkylene which may have substituents or benzylidene which may have substituents.

"Alkyl" means straight and branched alkyl residues of 1 to 10 carbon atoms. Examples of suitable alkyl residues include, but are not limited, to methyl, ethyl, diethyl, n-propyl, isopropyl, di-isopropyl, acetyl, n-butyl, isobutyl, set-butyl, tert-butyl, pentyl, hexyl, and the like.

"Alkylene" and "alkenylene" mean a -CₙH₂ₙ- or -CₙHₙ- straight or branched chain radical of n = 2-10, for example ethylene, propylene, butylene.

"Cycloalkyl" and "cycloalkylene" mean carbocyclic radicals of 3 to 8 carbon atoms.

"Aryl" means phenyl and naphthyl.

"Alkyloxy" and "aryloxy" mean a C₁-C₁₀-alkyl-O group and an aryl-O group.

"Heterocyclic" means a saturated or partially unsaturated 4-10 membered ring system in which one or more rings contain one or more heteroatoms selected from the group consisting of N, O or S;

"Aralkyl" means C1-4 alkyl as defined above attached to an aryl or heterocyclic moiety as defined above.

Substituents may be C1-C6 alkyl or alkoxy.

Ruthenium carbonyl pincer complexes can be easily prepared from ruthenium carbonyl precursors and the pincer ligands amino diphosphines. Pincer ligands amino diphosphines can be easily prepared by reaction of the corresponding phosphine compounds in the presence of a base and alkylamines. Inorganic ruthenium compounds like ruthenium carbonyl complexes are readily available precursors.

An especially preferred used catalyst of the present invention is a PNP pincer complex of ruthenium of the above formula I, wherein X and Y have the above meaning and L is of formula II, wherein Q¹ and Q² are identical and R¹, R², R³ and R⁴ are identical. Preferably Q¹ and Q² is identical and means C₂-C₄ alkylene more preferred C₂-alkylene. R¹, R², R³ and R⁴ are preferred identical a C2-C4 alkyl or aryl residue, preferably phenyl.

Especially preferred is a catalyst having the following formulae of compounds 1 and 2.

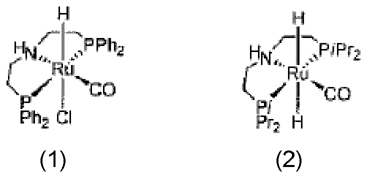

The inventive used catalyst *H*PNP^{Ph}Ru (1) is preferably added in the form of the pre-formed ruthenium-pincer ligand complex. The pre-formed ruthenium-pincer ligand complex is available commercially or may be prepared according to methods well known or, for example, by employing the procedure according to W02011048727.

*H*PNP^{*i*Pr}Ru (2) represents an expected *in situ* formed catalyst formed by mixing pincer ligand *H*PNP^{*i*Pr} 2a and metal precursor 2b.

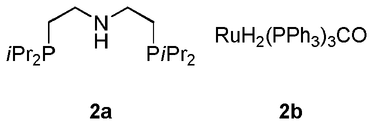

The inventive reaction to produce alkyl esters by dehydrogenation of a primary alcohol using an above catalyst can be carried out in the presence of a base, preferably in the presence of a metal alkoxide/alcoholate. Bases are known and can be preferably selected from the group comprising Na, K, NaH, NaOH, KOH, CsOH, LiOEt, NaOEt, KOEt, KO*^{t}*Bu, LiHCO₃, NaHCO₃, KHCO₃, CsHCO₃, Li₂CO₃, Na₂CO₃ K₂CO₃ and Cs₂CO₃, NaOEt is preferred.

The temperature employed during the dehydrogenation step is less than 200 °C, preferably between 20 and 150 °C. Preferable is a temperature of 40 - 90°C. This is a considerably lower reaction temperature than the temperature normally used (> 130°C) for acceptorless dehydrogenation of alcohols for ester synthesis by homogeneous catalysis. The reaction takes place in both an open system where the produced hydrogen gas is leaving the system and a closed system where the produced hydrogen gas stays in the reaction vessel, preferably open.

The reaction takes place with catalyst loading ≥25 ppm (0.0025 mol%), preferably with catalyst loading 50-500 ppm (0.0050 - 0.0500 mol%).

The used Ru-based pincer catalysts efficiently catalyze the direct formation of alkyl esters. Notably, neither solvent nor hydrogen acceptor are employed. An especially significant catalytic activity in the reaction is achieved utilizing aliphatic PNP pincer ligands on a Ru-based system

The invention refers especially to a process for producing ethyl acetate, which comprises reacting ethanol in the presence of an above ruthenium-based homogeneous catalyst system. The preferred use of NaOEt leads to the highest turnover frequency. Especially within the range of 0.3-1.3 mol% NaOEt with respect to ethanol, a fairly similar activity is observed.

Using a catalyst of compound (1) or (2) the catalyst activity proved to be steady over the range of at least 10 hours reaction time and up to approximately 90% conversion.

A first homogenously catalyzed acceptorless dehydrogenation was developed preferably the dehydrogenation of ethanol to ethyl acetate was successfully developed. The industrially important reaction allows for a cost-efficient synthesis of ethyl acetate. The turnover number (abbreviated *TON* and used as the number of moles of substrate that a mole of catalyst can convert before becoming inactivated) - applied catalyst loadings in the ppm-range allowed for a high yield of ethyl acetate with a TON exceeding 15.000. The turnover frequency (TOF = TON/h) can be about 1130/h and more.

Also other alcohols are applicable in the dehydrogenative esterification as well.

For example using 1-octanol shows a turnover frequency which is only lower by a factor of 1.5 compared to ethanol under the same conditions. By this procedure, octyl octanoate is formed.

The invention constitutes an important example of using renewable feedstock for more benign production of bulk chemicals.

### Example 1

Acceptorless dehydrogenation of ethanol to ethyl acetate according to the following scheme:

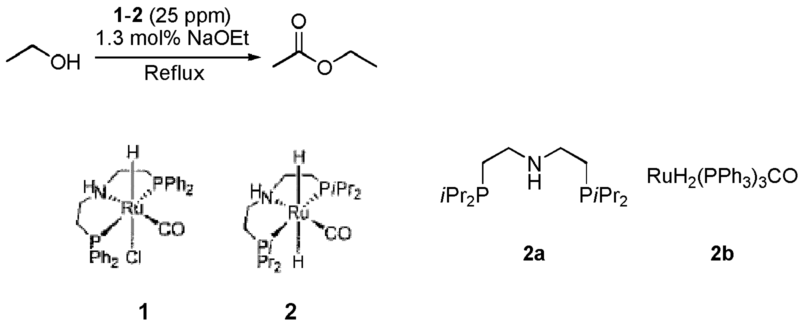

Catalyst compound 1 and the precurcors 2a and 2b for catalyst 2 were bought from fine chemical suppliers (Strem Chemicals) and used as received.

Ethanol was bought with highest purities and containing molsieves and was distilled over sodium prior to use. All substances were stored under argon atmosphere.

All experiments were carried out under argon atmosphere with the extrusion of air. If refluxing condition were desired, a 90 °C heating source was applied. For the TOF determinations, the volume of gas evolution was measured. This was done by manual burette measurements using a 500 mL or 1 L burette, respectively. The production of hydrogen as the primary gas evolved was verified by gas-phase GC. The yield of ethyl acetate was determined by liquid-phase GC using hexadecane as internal standard and toluene as diluent. Representative reactions were reproduced to ensure outcome validity.

Performed with one of the catalysts 1 or 2 (25 ppm, 4.2 µmol) and NaOEt (1.3 mol%, 2.2 mmol) in refluxing EtOH (10 mL, 171.3 mmol)) and internal standard (1 mL hexadecane).

TOF was determined by burette measurements after 2 hours of reaction time. For long-term reactions, an oil-trap between the condenser and burette can be employed.

| Entry | Catalyst [25 ppm] | TOF [h⁻¹]*^{b}* |
|---|---|---|
| 1 | 1 | 1134 |
| 2 | 2 | 1107 |

Mild conditions with refluxing ethanol in an open system (a 90 ° C heating source is applied) are employed.

The *H*PNP^{Ph} Ru complex 1 showed high activity with a TOF of 1134 h⁻¹ (Table 1, entry 1). The *in situ* system 2 comprising an *H*PNP^{*i*Pr} ligand coordinating to a RuH₂ type structure, showed an activity in the similar range as 1.

### Example 2

Optimizing ethyl acetate yield and catalyst turnover numbers (TON) using catalyst 1 Table 2 summarizes the results collected by varying the catalyst loading and temperature, and conducting the reaction until gas evolution has ceased.

Performed with catalyst 1 (amount given in Table 2) and NaOEt (amount given in Table 2) in EtOH (10 mL, 171.3 mmol) and internal standard (1 mL hexadecane). The reaction temperature is given in Table 2. An oil-trap was employed immediately after the condenser. All reactions were conducted until gas evolution has ceased (6-46h). The ethyl acetate yield is determined by GC.

**Table 2**

| Entry | Amount of cat. 1 [ppm] | Amount of NaOEt [mol %] | Temp. [°C] | Yield [%] | TON |
|---|---|---|---|---|---|
| 1 | 500 | 1.3 | Reflux (90) | 81 | 1620 |
| 2 | 500 | 1.3 | 70 | 70 | 1400 |
| 3 | 50 | 0.6 | Reflux (90) | 77 | 15400 |

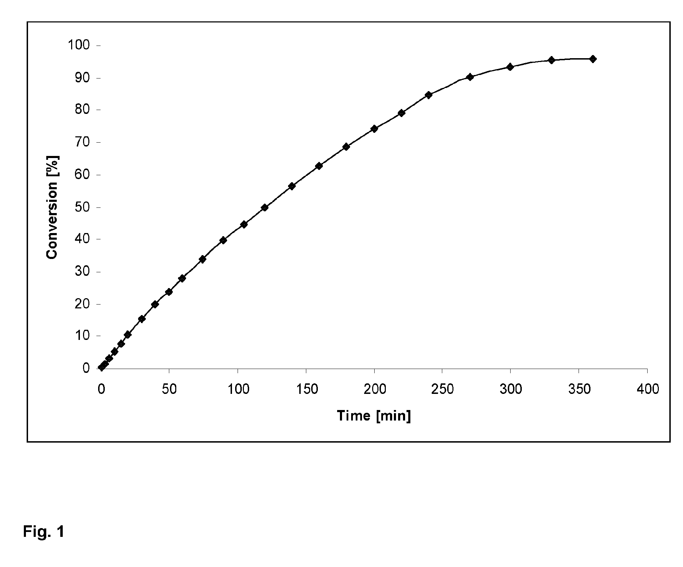

Increasing the catalyst loading from 50 to 500 ppm, and conducting the reaction until gas evolution ceased, led to a 81% yield of ethyl acetate with a TON of 1620. Evolved gas volume determined by burette measurements. The reaction was conducted for 6h, by which the gas evolution had ceased (see Fig. 1).

An attempt to lower the reaction temperature resulted in a slightly inferior yield and TON (70% and 1400, respectively). As it can be seen the reaction can be performed at sub-refluxing temperatures.

This is the first example hereof for acceptorless dehydrogenation of ethanol to form ethyl acetate. The catalyst is still active at lower temperatures, e.g. at 60 °C.

Employing 50 ppm of 1 led to a 77% yield of ethyl acetate with a TON of 15400. The TOF values for this entry were observed to be almost constant for the first 10 hours of reaction time with a TOF (2h) of 934 h⁻¹ and TOF (10h) of 730 h⁻¹. This slight diminishing activity is due to the exhaustion of ethanol during the reaction course, and ethyl acetate formation does not impede the reaction nor deactivates the catalyst over time.

Evolved gas volume determined by burette measurements. The reaction was conducted for 46h, by which the gas evolution had ceased, and the yield was 77%.

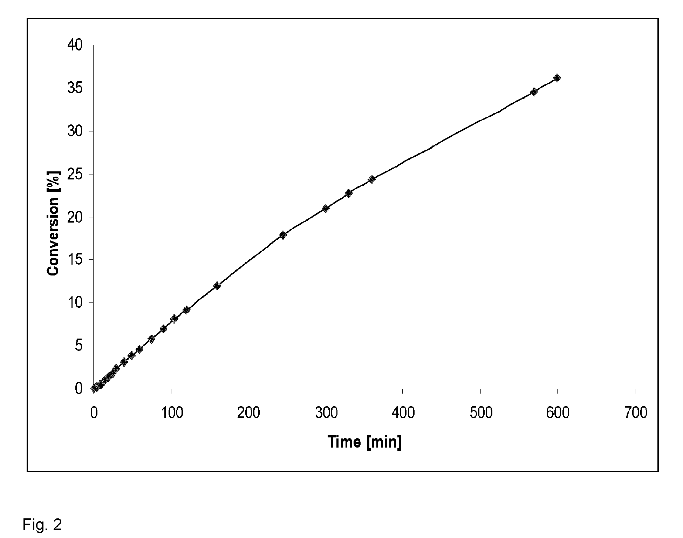

In Fig. 2 is shown ethanol conversion according to gas evolution measurements for the first 10h of the reaction.

### Example 3

Employing 500 ppm of 1 in 1-octanol instead of ethanol led to the formation of octyl octanoate. The TOF(2h) is 330 h⁻¹. This ca. 1.5 lower activity compared to ethanol is expected to be due to a lower hydroxy/carbon-chain ratio in 1-octanol.

## Claims

1. A process for producing alkyl esters, which comprises reacting a primary alcohol in the presence of a ruthenium-based (Ru-based) homogeneous catalyst system comprising a tridentate pincer ligand.

2. Process according claims 1, wherein the Ru-based catalyst complex has the following general formula **I**
RuXY(CO)L, I
wherein
X = hydride or halogen
Y = hydride or halogen
L = tridentate pincer ligand, selected from a PNP pincer type complex, a PNN pincer type complex, a PCP pincer type complex, a NCN pincer type complex, a PCN type complex or a NNN pincer type complex.

3. Process according to any of claims 1 or 2, wherein the catalyst complex is an PNP pincer complex of ruthenium of formula I
wherein
X and Y have the meaning as above defined and
L is of formula II 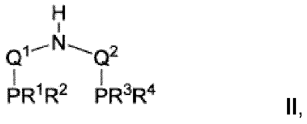 wherein
Q¹, Q² are indentical or different selected from the group consisting of divalent alkylene (-CₙH₂ₙ-; n = 2 -10) or alkenylene (-CₙHₙ-; n = 2-10) which may have substituents, cycloalkylene (C₂-C₁₀) which may have substituents or benzylidene which may have substituents;
R¹, R², R³, R⁴ may be identical or different and represent hydrogen, alkyl, cycloalkyl, aryl, aralkyl, an alkyloxy group, a cycloalkyloxy group, an aryloxy group, aralkyloxy group, a heterocyclic group, amino group or substituted; R⁴ or R³ and R¹ and R², these are not to form a ring with the phosphorus atom adjacent to bond together may be.

4. Process according to claim 5, wherein the residue L is of formula II, wherein Q¹ and Q² are identical and mean C₂-C₄ alkylene, more preferred C₂-alkylene, and R¹, R², R³ and R⁴ are identical a C₂-C₄ alkyl or an aryl residue, preferably phenyl.

5. Process according to any of claims 1 to 6, wherein the used catalyst is a Ru-based catalyst complex having the formula (1) or (2)

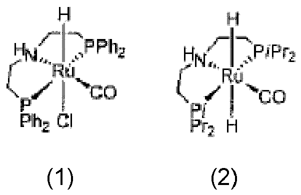

6. Process according to any of claims 1 to 5, wherein the primary alcohol is a C₂ to C₁₈ alcohol.

7. Process according to any of claims 1 to 6, wherein the primary alcohol is selected from the group consisting of ethanol, 1-propanol, 1-butanol, 1-octanol and 1-octadecanol, preferably ethanol or 1-octanol.

8. Process according to any of claims 1 to 7, wherein no solvent and no hydrogen acceptor is used.

9. Process according to any one of claims 1 to 8, wherein the reaction is in presence of a base, preferably in presence of a metal alkoxide/alcoholate.

10. Process according to claim 9, wherein the base is selected from the group consisting of Na, K, NaH, NaOH, KOH, CsOH, LiOEt, NaOEt, KOEt, KO*^{t}*Bu, LiHCO₃, NaHCO₃, KHCO₃, CsHCO₃, Li₂CO₃, Na₂CO₃ K₂CO₃ and Cs₂CO₃, preferably NaOEt.

11. Process according to any one of claims 1 to 10, wherein the temperature employed during the dehydrogenation step is less than 200 °C, preferably between 20 - 150°C.

12. Process according to any one of claims 1 to 11, wherein the reaction performs with catalyst loading below 500 ppm.

13. Use of a Ru-based catalyst complex of formula (1) or (2) 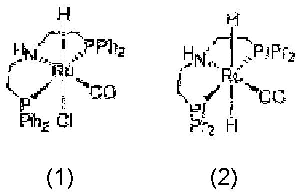 for producing an alkyl ester, by reacting the corresponding alcohol in presence of one of the said catalysts or precursors thereof at a temperature of 20 to 150°C, whereby no solvent and no hydrogen acceptor is used.

14. Use according to claim 13, for producing ethyl acetate by reacting ethanol at a temperature of 40 to 90°C.
